# EUROPEAN PATENT SPECIFICATION

(11) **EP 3 015 045 B1**
(45) Date of publication and mention of the grant of the patent: **01.08.2018**
(21) Application number: 14816918.8
(22) Date of filing: 10.06.2014
(51) Int. Cl.: A61B 1/00, G02B 23/24

(54) **SPIRAL UNIT AND GUIDE DEVICE**
SPIRALEINHEIT UND FÜHRUNGSVORRICHTUNG
UNITÉ SPIRALÉE ET DISPOSITIF DE GUIDAGE

(30) Priority: 26.06.2013 US 201361839433 P
(43) Date of publication of application: 04.05.2016
(73) Proprietor: Olympus Corporation, Tokyo 192-8507 (JP)
(72) Inventor: AILINGER, Robert E., Massachusetts 01772-2104 (US); FRASSICA, James J., Massachusetts 01772-2104 (US); ANDREWS, Richard M., Massachusetts 01772-2104 (US); MIYOSHI, Hiroaki, Tokyo 192-8507 (JP)
(74) Representative: Gunzelmann, Rainer
(86) International application number: PCT/JP2014/065377
(87) International publication number: WO 2014/208333

(56) References cited:
- EP-A1- 2 915 475
- WO-A1-2012/137363
- JP-A- 2009 291 550
- US-A1- 2013 035 552

## Description

### Technical Field

The present invention relates to a spiral unit that can rotate to an insertion section in a state where the insertion section having a longitudinal axis is inserted thereinto, and an introduction apparatus having the spiral unit for various kinds of ducts.

### Background Art

For example, US 2012/0029281 A1 discloses a spiral unit which inserts an insertion section. The spiral unit can be rotated in two directions around a longitudinal axis (a circumferential direction) of the insertion section. Thus, when the spiral unit is appropriately rotated to the insertion section, a distal end of the insertion section can be moved to a far side and a near side of a duct.

For example, at the time of rotating the spiral unit in US 2012/0029281 A1 to the insertion section, and moving and inserting the distal end portion of the insertion section to the far side in the duct, or moving the same to the near side to remove the same therefrom, an end of the spiral unit is caught on an inner peripheral surface of the duct due to steps between a distal end and a proximal end of the spiral unit and an outer peripheral surface of the insertion section in some cases.

As a further example, US 2013/035552 A1 discloses an endoscope including a flexible portion extended to a part on a proximal direction side of an insertion main body along a longitudinal axis, and a passive bending portion provided to a distal direction side of the flexible portion in the insertion main body, the passive bending portion being more flexible than the flexible portion and configured to passively bend when external force acts thereon.

The endoscope of US 2013/035552 A1 further comprises a spiral attachment unit according to the preamble of claim 1, which includes a fin portion spirally extended along the longitudinal axis, and through which the insertion section is inserted, and a holding portion provided to the insertion main body, and configured to hold the attachment unit in a state that the attachment unit covers the passive bending portion and that the attachment unit is rotatable about the longitudinal axis with respect to the insertion main body.

### Summary of Invention

It is an object of the present invention to provide a
spiral unit that can prevent an end thereof from being caught on an inner peripheral surface of a duct in a state of being disposed on an insertion section, and an introduction apparatus having the spiral unit for various kinds of ducts.

According to one aspect of the present invention, a spiral unit into which an insertion section of an introduction apparatus having a longitudinal axis is to be inserted, and is rotatable to the insertion section in a state of being disposed on the insertion section, the spiral unit includes the features of claim 1. A spiral unit may include: a tube body which is configured to be arranged along the longitudinal axis, and has a spiral fin arranged on an outer peripheral surface thereof; a taper section which is a tubular shape and which is provided on at least one of a distal end side and a proximal end side of the tube body along the longitudinal axis, has a proximal part close to the tube body and a distal part apart from the tube body, has an outer periphery that is diameter-reduced toward the longitudinal axis more at the distal part than at the proximal part along the longitudinal axis, and exerts a diameter reducing force to an outer peripheral surface of the insertion section at the distal part in the range where sliding is possible in a periaxial direction of the longitudinal axis; and a reduced diameter regulator which is removably arranged in the taper section, holds a state of expanding an inner diameter of the taper section so that insertion of the insertion
section into the taper section is allowed, to regulate reduction of the inner diameter of the taper section, and is removed from the taper section along with the insertion of the insertion section into the taper section, thereby exerting the diameter reducing force to the taper section more at the distal part than at the proximal part on the outer peripheral surface of the insertion section in the range where sliding is possible in the periaxial direction of the longitudinal axis.

### Brief Description of Drawings

FIG. 1 is a schematic view showing an endoscope (an introduction apparatus) mounting a spiral unit according to first and second embodiments disposed thereto, and its peripheral unit;
FIG. 2 is a longitudinal sectional view schematically showing a configuration of a second relay connecting section of an insertion section of the endoscope mounting the spiral unit according to the first embodiment disposed thereto;
FIG. 3 is a schematic transverse sectional view taken along a line III in FIG. 2, showing the second relay connecting section of the insertion section of the endoscope assembling the spiral unit according to the first embodiment disposed thereto;
FIG. 4 is a schematic transverse sectional view taken along a line IV-IV in FIG. 2, showing the second relay connecting section of the insertion section of the endoscope assembling the spiral unit according to the first embodiment disposed thereto;
FIG. 5A is a schematic perspective view showing a proximal side taper section in a state that a diameter of a proximal end of a tube body of the spiral unit according to the first embodiment and a diameter of a distal part to the tube body are expanded by a reduced diameter regulator (an expanded diameter holder);
FIG. 5B is a schematic perspective view showing the proximal side taper section in a state that the reduced diameter regulator (the expanded diameter holder) depicted in FIG. 5A is removed from the proximal end of the tube body of the spiral unit according to the first embodiment and the distal part to the tube body to reduce the diameters;
FIG. 6A is a schematic view showing a state that the insertion section is inserted into the spiral unit in a state that the reduced diameter regulator (the expanded diameter holder) is disposed to the distal part to the tube body in the proximal side taper section of the spiral unit according to the first embodiment;
FIG. 6B is a schematic view showing a state that a breaking part of a ring-shaped member of the reduced diameter regulator is broken to remove the reduced diameter regulator from the distal part to the tube body in the proximal side taper section of the spiral unit according to the first embodiment, and a proximal end of the proximal side taper section (the distal part to the tube body) abuts on an outer peripheral surface of the insertion section to be slidable in a periaxial direction (a circumferential direction) of a longitudinal axis;
FIG. 7A is a schematic view showing a state that the insertion section is inserted into the spiral unit while the reduced diameter regulator (the expanded diameter holder) is disposed to the distal part to the tube body in the proximal side taper section of the spiral unit according to the first embodiment;
FIG. 7B is a schematic view showing a state that the reduced diameter regulator (the expanded diameter holder) is removed from the distal part to the tube body in the proximal side taper section of the spiral unit according to the first embodiment to separate end portions of the ring-shaped member from each other, and the proximal end of the proximal side taper section (the distal part to the tube body) abuts on the outer peripheral surface of the insertion section to be slidable in the periaxial direction (the circumferential direction) of the longitudinal axis;
FIG. 8 is a schematic transverse sectional view of the second relay connecting section of the insertion section, which is a modification of the insertion section of the endoscope mounting the spiral unit according to the first embodiment disposed thereto;
FIG. 9A is a schematic top view showing a part of the proximal end of the tube body of the spiral unit and a part of the proximal side taper section according to the modification of the first embodiment;
FIG. 9B is a schematic cross-sectional view taken along a line 9B-9B in FIG. 9A, showing a part of the proximal end of the tube body of the spiral unit and a part of the proximal side taper section according to the modification of the first embodiment; and
FIG. 10 is a schematic partial cross-sectional view showing a proximal end of a tube body of a spiral unit and a proximal side taper section according to a second embodiment.

### Description of Embodiments

Modes for carrying out the present invention will now be described hereinafter with reference to the drawings.

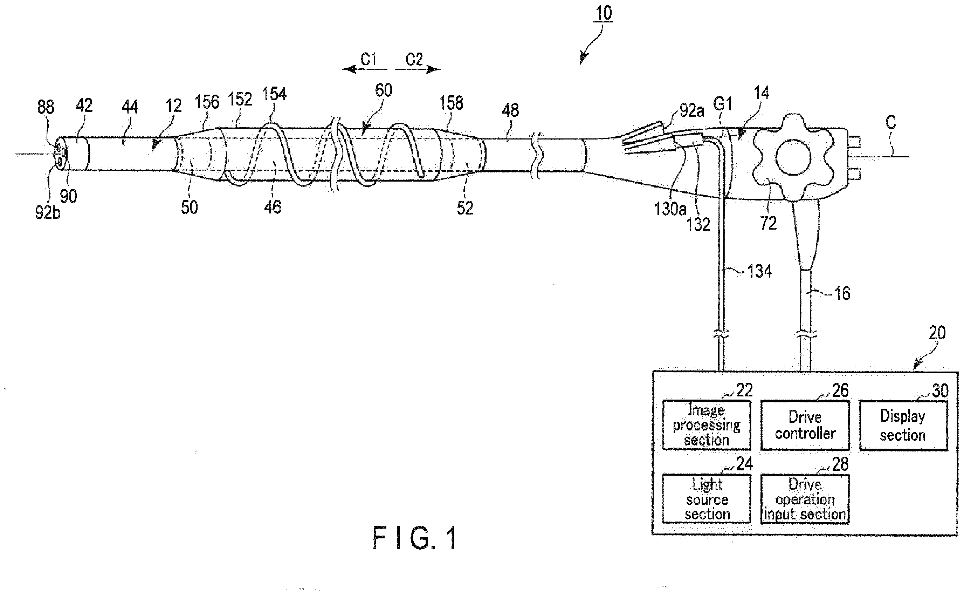

A first embodiment will be described with reference to FIG. 1 to FIG. 9B.

As shown in FIG. 1, an endoscope (an introduction apparatus for various kinds of ducts) 10 has a longitudinal axis (a central axis) C. One of directions parallel to the longitudinal axis C (a direction of an arrow C1 in FIG. 1) is a distal end direction, and a direction opposite to the distal end direction C1 (a direction of an arrow C2 in FIG. 1) is a proximal end direction. The endoscope 10 includes an insertion section (an endoscope insertion section) 12 extended along the longitudinal axis C, an operation section (an endoscope operation section) 14 provided on a proximal end direction side of the insertion section 12, and a spiral unit 60 mounted on an outer periphery of the insertion section 12. The insertion section 12 is extended along the longitudinal axis C, and is inserted into a duct from a distal end thereof at the time of using the endoscope 10.

A universal cable 16 is extended from the operation section 14. A distal end of the universal cable 16 to the operation section 14 can be connected to a peripheral unit 20. The peripheral unit 20 includes, e.g., an image processing section 22, a light source section 24, a drive controller 26, a drive operation input section 28, and a display section 30.

The insertion section 12 includes a distal rigid section 42 provided at a region on the most distal side, a bending section 44 provided on a proximal end direction side of the distal rigid section 42, a first flexible section 46 provided on the proximal end direction side of the bending section 44, and a second flexible section 48 provided on the proximal end direction side of the first flexible section 46. The bending section 44 and the first flexible section 46 are connected to each other through a first relay connecting section 50. The first flexible section 46 and the second flexible section 48 are connected to each other through a second relay connecting section 52.

The spiral unit 60 is extended along the longitudinal axis C between, e.g., the first relay connecting section 50 and the second relay connecting section 52. The spiral unit 60 is disposed to the insertion section 12 in a state that the insertion section 12 is inserted in the spiral unit 60. In this embodiment, the spiral unit 60 can rotate in a periaxial direction of the longitudinal axis C to the insertion section 12.

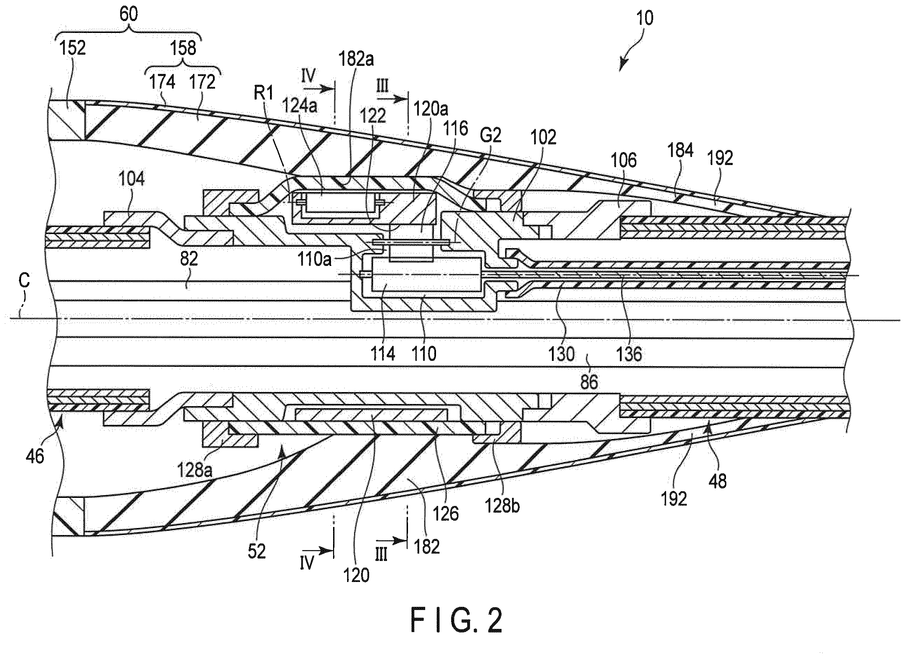

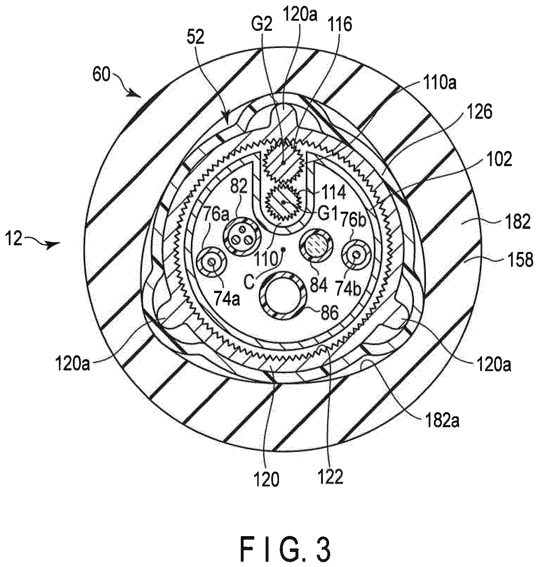

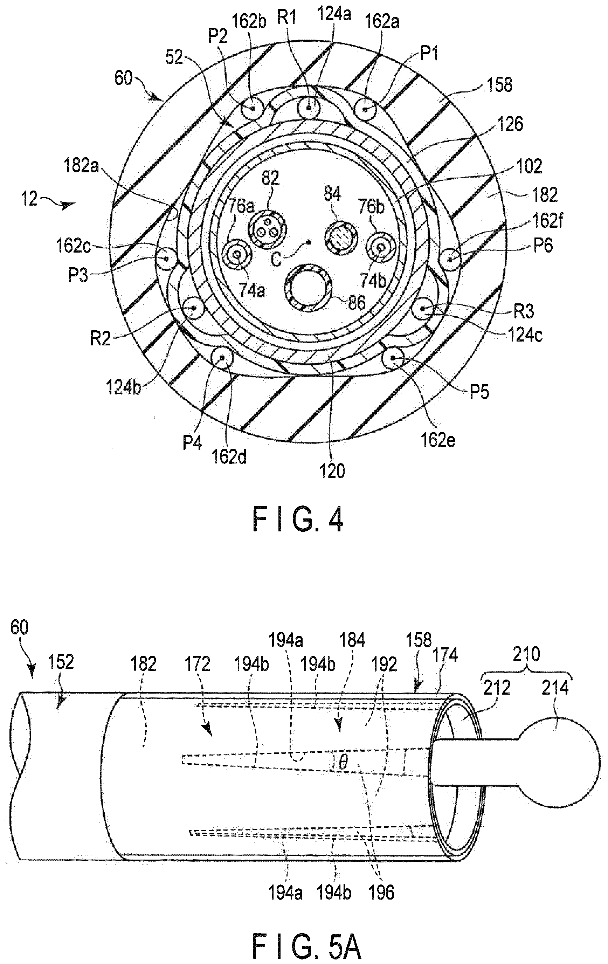

FIG. 2 shows a configuration of the second relay connecting section 52. FIG. 3 is a transverse sectional view taken along a line III-III in FIG. 2, and FIG. 4 is a transverse sectional view taken along a line IV-IV in FIG. 2.

As shown in FIG. 1, a bending operation knob 72 which is a bending operation input section to which a bending operation of the bending section 44 is input is provided on an outer surface of the operation section 14. As shown in FIG. 3 and FIG. 4, in the insertion section 12, bending wires 74a and 74b, and coils 76a and 76b inserting the bending wires 74a and 74b respectively are extended along the longitudinal axis C. Distal ends of the coils 76a and 76b are connected to, e.g., an inner peripheral surface of the first relay connecting section 50, respectively. In the operation section 14, proximal ends of the bending wires 74a and 74b are connected to a pulley (not shown) coupled with the bending operation knob 72, respectively. Distal ends of the bending wires 74a and 74b are connected to, e.g., a distal end section of the bending section 44. The bending wire 74a or the bending wire 74b is pulled by an operation of the bending operation knob 72, and the bending section 44 bends in a desired direction.

It is to be noted that, in this embodiment, the two bending wires 74a and 74b are provided, and the bending section 44 can bend in two directions but, for example, four bending wires may be provided, and the bending section 44 can thereby bend in four directions.

As shown in FIG. 2 to FIG. 4, an observation optical system, an illumination optical system, and a channel are arranged in the insertion section 12. More specifically, in the insertion section 12, an imaging cable 82, a light guide 84, and a treatment tool channel tube 86 are extended along the longitudinal axis C. In the distal rigid section 42 (a distal end portion of the insertion section 12), an imaging element (not shown) that images a subject is provided. The imaging element images the subject through an observation window 88. A distal end of the imaging cable 82 is connected to the imaging element. The imaging cable 82 is extended in the insertion section 12, the operation section 14, and the universal cable 16, and a proximal end thereof is connected to the image processing section 22 in the peripheral unit 20. The image processing section 22 generates an image of the subject. The generated image of the subject is displayed in the display section 30.

The light guide 84 is extended in the insertion section 12, the operation section 14, and the universal cable 16, and a proximal end thereof is connected to the light source section 24 in the peripheral unit 20. Light exiting from the light source section 24 is guided by the light guide 84, and applied to the subject from an illumination window 90 in the distal end portion (the distal rigid section 42) of the insertion section 12.

As shown in FIG. 1, a treatment tool insertion section 92a into which a treatment tool such as a forceps is inserted is provided on the outer surface of the operation section 14. A proximal end of the treatment tool channel tube 86 is connected to the treatment tool insertion section 92a through the inside of each of the insertion section 12 and the operation section 14. The treatment tool inserted from the treatment tool insertion section 92a protrudes from an opening portion 94 of the distal rigid section 42 toward the distal end direction through the inside of the treatment tool channel tube 86. Further, in a state that the treatment tool protrudes from the opening portion 92b of the distal rigid section 42, a treatment is given by the treatment tool.

As shown in FIG. 2, the second relay connecting section 52 has a base member 102. A proximal end portion of the first flexible section 46 is coupled with a distal end portion of the base member 102 through a relay member 104. Thus, the first flexible section 46 is coupled with the second relay connecting section 52. A distal end portion of the second flexible section 48 is coupled with a proximal end portion of the base member 102 through a relay member 106. Thus, the second flexible section 48 is coupled with the second relay connecting section 52.

As shown in FIG. 2 to FIG. 4, the second relay connecting section 52 has a hollow portion 110 in the base member 102. The hollow portion 110 is opened to the outside in an opening portion 110a. Furthermore, a drive gear 114 and a relay gear 116 are disposed to the base member 102. The drive gear 114 is arranged in the hollow section 110, and the relay gear 116 is arranged near the opening portion 110a of the hollow portion 110. The drive gear 114 is meshed with the relay gear 116. The drive gear 114 can rotate around a drive axis G1. The relay gear 116 can rotate around a gear axis G2.

A rotary tubular member 120 formed into a tubular shape is disposed to the base member 102 of the second relay connecting section 52. The rotary tubular member 120 can rotate in the periaxial direction of the longitudinal axis C to the insertion section 12 (the base member 102). An inner peripheral gear section 122 is arranged over the entire inner peripheral surface of the rotary tubular member 120 in the periaxial direction of the longitudinal axis C. The inner peripheral gear section 122 of the rotary tubular member 120 is meshed with the relay gear 116.

The rotary tubular member 120 has a roller support section 120a that supports, e.g., three inner rollers 124a, 124b, and 124c. The inner rollers 124a, 124b, and 124c are arranged at substantially equal intervals in the periaxial direction (the circumferential direction) of the longitudinal axis C. The inner rollers 124a, 124b, and 124c have corresponding roller axes R1, R2, and R3, respectively. The inner rollers 124a, 124b, and 124c can rotate around the corresponding roller axes R1, R2, and R3 to the rotary tubular member 120, respectively. Moreover, the inner rollers 124a, 124b, and 124c can rotate in the periaxial direction of the longitudinal axis C together with the rotary tubular member 120 to the insertion section 12 (the base member 102).

Outer sides of the rotary tubular member 120 and the inner rollers 124a, 124b, and 125c are covered with a tubular cover member 126. A distal end of the cover member 126 is fixed to the base member 102 by an annular locking member 128a. The distal end of the cover member 126 is liquid-tightly maintained between the base member 102 and the cover member 126 by the locking member 128a. A proximal end of the cover member 126 is fixed to the base member 102 by an annular locking member 128b. The proximal end of the cover member 126 is liquid-tightly maintained between the base member 102 and the cover member 126 by the locking member 128b. Thus, a liquid is prevented from entering the hollow portion 110, the rotary tubular member 120, and the inner rollers 124a, 124b, and 124c placed on the inner side of the cover member 126.

As shown in FIG. 3 and FIG. 4, the cover member 126 outwardly protrudes in regions where the inner rollers 124a, 124b, and 124c are placed in the periaxial direction of the longitudinal axis C, respectively. It is to be noted that the cover member 126 is fixed on the outer side of the base member 102, namely, fixed on the outer periphery of the insertion section 12, whereas the rotary tubular member 120 can rotate in the periaxial direction of the longitudinal axis C to the cover member 126.

As shown in FIG. 1, the operation section 14 has a proximal opening 130a of a channel 130 into which a later-describe drive shaft 136 is inserted on the outer surface thereof. A motor 132 which is a drive member is disposed to the proximal opening 130a of the channel 130. One end of a motor cable 134 is connected to the motor 312. The other end of the motor cable 134 is connected to the drive controller 26 in the peripheral unit 20.

As shown in FIG. 2, the drive shaft 136 which is a linear member is extended along the drive axis G1 in the second flexible section 48 of the insertion section 12. A distal end of the drive shaft 136 is connected to the drive gear 114. A proximal end of the drive shaft 136 is connected to the motor 132 disposed in the proximal opening 130a of the channel 130. Additionally, a distal end of the channel 130 is connected to the base member 102 to communicate with the hollow portion 110. A proximal end of the channel 130 is connected to the proximal opening 130a. The drive shaft 136 is extended through the inside of the channel tube 130.

The drive controller 26 supplies electric power to the motor 132 through the motor cable 134 by an operation input of the drive operation input section 28, and executes drive control over the motor 132. The drive controller 26 drives the motor 132, and thereby generates a rotary drive force to rotate the drive shaft 136 to the drive shaft 136. Thus, the drive shaft 136 and the drive gear 14 rotate around the drive axis G1. Here, the drive axis G1 runs through the center of the drive gear 114 and the drive shaft 136, and is substantially parallel to the longitudinal axis C in the second flexible section 48. Further, the drive shaft G1 bends toward the proximal opening 130a of the channel 130 in the operation section 14.

When the drive gear 114 rotates around the drive axis G1, the relay gear 116 meshed with the drive gear 114 rotates around the gear axis G2. The rotary tubular member 120 rotates in the periaxial direction of the longitudinal axis C by the inner peripheral gear section 122 meshed with the relay gear 116. That is, rotary drive force of the motor 132 is transmitted to the drive shaft 136, the drive gear 114, the relay gear 116, and the rotary tubular member 120. Thus, when the rotary tubular member 120 rotates in the periaxial direction of the longitudinal axis C, the inner rollers 124a, 124b, and 124c supported by the rotary tubular member 120 move in the periaxial direction of the longitudinal axis C to the insertion section 12 and the cover member 126.

As shown in FIG. 1, the spiral unit 60 has a tube body 152 arranged with a spiral fin 154 disposed on an outer peripheral surface thereof, a distal side taper section 156 having a tubular shape provided on a distal end side of the tube body 152, and a proximal side taper section 158 having a tubular shape provided on a proximal end side of the tube body 152.

The spiral unit 60 according to this embodiment is disposable. Thus, the spiral unit 60 is disposed on the outer periphery of the first flexible section 46 of the insertion section 12 and used in this state every time the endoscope 10 is used. After the use of the endoscope 10, the spiral unit 60 is broken, removed from the outer side of the insertion section 12, and discarded.

The tube body 152 assembling the spiral fin 154 disposed thereon is made of, e.g., a thermoplastic resin. The spiral fin 154 is provided along a fin axis F spirally extended around the longitudinal axis C. An inner periphery of the tube body 152 is formed to allow insertion of the distal rigid section 42, the bending section 44, and the first flexible section 46 of the insertion section 12.

The distal side taper section 156 is formed into a taper shape so that its outer diameter is reduced toward the distal end direction side (the outer diameter is reduced from a proximal part to a distal part of the tube body 152). The proximal side taper section 158 is formed into a taper shape so that its outer diameter is reduced toward the distal end direction side (the outer diameter is reduced from the proximal part to the distal part of the tube body 152). Although the particulars will be described later, the distal side taper section 156 and the proximal side taper section 158 are formed so that the spiral unit 60 can exert a diameter reducing force in the range where it can slide at a distal part to the tube body 152 when it rotates in the periaxial direction (the circumferential direction) of the longitudinal axis C on the outer peripheral surface of the insertion section 12.

As shown in FIG. 4, the spiral unit 60 has outer rollers 162a, 162b, ..., and 162f, which are engaged to transmit a rotational force in the periaxial direction of the longitudinal axis C of the insertion section 12 to the tube body 152, on its inner peripheral surface. Specifically, the six outer rollers 162a, 162b, ..., and 162f are disposed on the inner peripheral surface (e.g., an inner peripheral surface 182a of a later-described annular portion 182) of the proximal side taper section 158. The outer rollers 162a, 162b, ..., and 162f are arranged on the outer side of the cover member 126. Along the periaxial direction (the circumferential direction) of the longitudinal axis C, the inner roller 124a is arranged between the two outer rollers 162a and 162b, and the inner roller 124b is arranged between the outer rollers 162c and 162d. Furthermore, along the periaxial direction (the circumferential direction) of the longitudinal axis C, the inner roller 124c is arranged between the outer rollers 162e and 162f. The outer rollers 162a, 162b, ..., and 162f have corresponding roller axes P1, P2, ..., and P6, respectively. The outer rollers 162a, 162b, ..., and 162f can rotate around the corresponding roller axes P1, P2, ..., and P6 to the cover member 126 and the proximal side taper section 158, respectively.

In this embodiment, the inner peripheral surface of the proximal side taper section 158 is formed into a shape other than a circle (a non-circular shape). Moreover, on the inner peripheral surface 182a of the proximal side taper section 158 having the outer rollers 162a, 162b, ..., and 162f disposed on the inner peripheral surface thereof, the inner rollers 124a, 124b, and 124c are supported on the outer side, and the rotary tubular member 120 having the cover member 126 fixed on the outer side thereof is fitted in the periaxial direction (the circumferential direction) of the longitudinal axis C.

It is to be noted that a positional relationship between the respective rollers 124a to 124c and 162a to 162f is not restricted to that described above. For example, it is also preferable to arrange the inner roller 124b or the inner roller 124c between the outer rollers 162a and 162b, to arrange the inner roller 124c or the inner roller 124a between the outer rollers 162c and 162d, and to arrange the inner roller 124a or the inner roller 124b between the outer rollers 162e and 162f.

Thus, when the rotary tubular member 120 rotates by driving of the motor 132 as described above, the inner roller 124a presses the outer roller 162a or the outer roller 162b in accordance with the rotating direction. Likewise, the inner roller 124b presses the outer roller 162c or the outer roller 162d, and the inner roller 124c presses the outer roller 162e or the outer roller 162f. Thus, the rotary driving force of the motor 132 is transmitted from the inner rollers 124a, 124b, and 124c to the outer rollers 162a to 162f, namely, transmitted to the spiral unit 60. Therefore, the spiral unit 60 including the tube body 152 having the fin 154 disposed thereon rotates in the periaxial direction of the longitudinal axis C to the insertion section 12 and the cover ember 126.

Thus, the outer rollers 162a, 162b, ..., and 162f can rotate together with the spiral unit 60 in the periaxial direction of the longitudinal axis C to the insertion section 12 (the base member 102).

It is to be noted that the inner rollers 124a, 124b, and 124c rotate around the corresponding roller axes R1, R2, and R3, respectively. Thus, friction between the respective inner rollers 124a, 124b, and 124c and the inner peripheral surface of the cover member 126 is reduced. Likewise, the outer rollers 162a, 162b, ..., and 162f rotate around the corresponding roller axes P1, P2, ..., and P6 respectively, and hence friction between the respective outer rollers 162a, 162b, ..., and 162f and the outer peripheral surface of the cover member 126 is reduced. Thus, the rotary driving force is appropriately transmitted to the spiral unit 60 from the inner rollers 124a, 124b, and 124c supported by the rotary tubular member 120, and the spiral unit 60 appropriately rotates to the base member 102 included in the second relay connecting section 52 of the insertion section 12. When the spiral unit 60 (the tube body 152 and the fin 154) rotates to the insertion section 12 in a state that the spiral fin 154 abuts on a wall portion such as an inner wall of a duct, a propulsive force to the distal end direction C1 or the proximal end direction C2 acts on the insertion section 12 mounting the spiral unit 60 disposed thereon along the longitudinal axis C.

Here, a configuration of the proximal side taper section 158 of the spiral unit 60 will now be described with reference to FIG. 2 to FIG. 5B. It is to be noted that, although a detailed description will be omitted, it is preferable to form the distal side taper section 156 into the same configuration as the proximal side taper section 158. That is, it is preferable to form the taper sections having the same configuration on the distal end side and the proximal end side of the tube body 152. That is, the taper section is provided on at least one of the distal end side and the proximal end side of the tube body 152 along the longitudinal axis C and has a proximal part close to the tube body 152 and a distal part apart from the same, a diameter of the outer periphery of the taper section is reduced along the longitudinal axis C at the distal part rather than the proximal part, and a diameter reducing force is exerted to the outer peripheral surface of the insertion section 12 at the distal part in the range where sliding in the periaxial direction of the longitudinal axis C is possible.

In this embodiment, the proximal side taper section 158 is formed into a double layer having an inner layer 172 and an outer layer (an outer jacket) 174. The inner layer 172 may be made of a resin material or made of a metal material as long as it is elastically deformable. It is desirable for the inner layer 172 to have electrical insulating properties. The outer layer 174 is formed into a cylindrical shape by using an elastic member such as a resin having stretch properties in a radial direction. The outer layer 174 is formed to cover the inner layer 172, namely, the entire outer periphery from distal ends to proximal ends of the later-described annular portion 182 and a deforming portion 184 (extended portions 192).

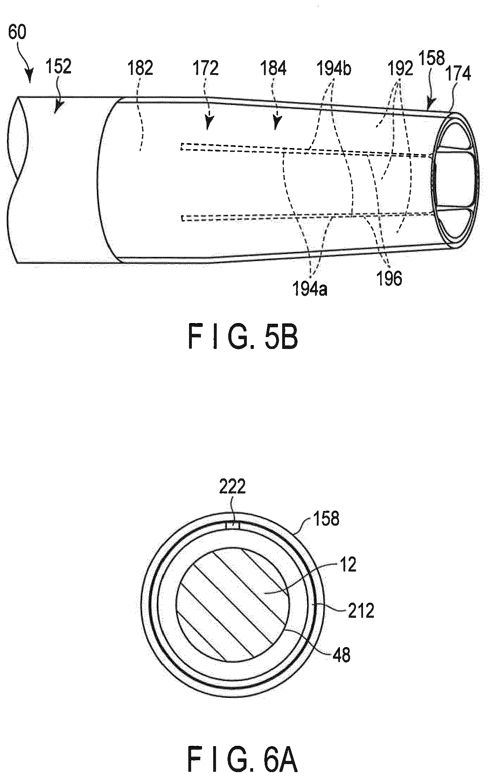

As shown in FIG. 5A and FIG. 5B, the inner layer 172 has the annular portion 182 coupled with the proximal end of the tube body 152 and the deforming portion 184 as an elastic member that is extended to the proximal end side from the annular portion 182 and elastically deformable. As a configuration that couples a distal end of the annular portion 182 (the proximal part to the tube body 152) with a proximal end of the tube body 152, for example, an adhesive or fitting can be appropriately used.

The deforming portion 184 has the extended portions 192 extended from the annular portion 182 to the proximal end side (the distal part to the tube body 152), respectively. Each extended portion 192 is formed into a strip shape. The extended portions 192 are aligned in the circumferential direction of the annular portion 182 (the circumferential direction of the longitudinal axis C). Each extended portion 192 has a pair of edge portions 194a and 194b. Each slit 196 is formed of the edge portions 194a and 194b between the respective extended portions 192. It is preferable to form each extended portion 192 in such a manner that a circumferential width of its proximal end side (a side apart from the annular portion 182), i.e., the distal part to the tube body 152 is gradually reduced as compared with its distal end side (a side close to the annular portion 182), i.e., the proximal part to the tube body 152. That is, the edge portions 194a and 194b form an opening angle θ of the slit 196 along the longitudinal axis C from the proximal part to the tube main body 152 toward the distal part to the tube main body 152. The edge portions 194a and 194b of the extended portions 192 adjacent to each other in the extended portions 192 are caused to abut on each other by an energizing force applied in a direction toward the longitudinal axis C by the outer layer 174. That is, the opening angle θ of each slit 196 is 0. Thus, a diameter of the deforming portion 184 can be reduced more on the proximal end side (the distal part) than on the distal end side (the proximal part) to the tube body 152. Here, the diameter of the diameter changing portion 184 can be reduced to an arbitrary diameter by adjusting the opening angle θ of each slit 196. On the other hand, when the edge portions 194a and 194b of the extended portions 192 adjacent to each other are separated from each other against the energizing force of the outer layer 174, the diameter can be expanded equally on both the proximal end side (the distal part) and distal end side (the proximal part) to the tube body 152. Thus, as a whole, the deforming portion 184 can be greatly displaced so that its proximal end side (the distal part to the tube body 152) can be moved closer to or away from the longitudinal axis C as compared with the distal end side (the proximal part to the tube body 152) by using the extended portions 192.

In addition, it is also preferable to form each extended portion 192 to have the energizing force so that its proximal end side can move closer to the central axis of the annular portion 182, i.e., the longitudinal axis C based on characteristics of its material. The extended portions 192 themselves are formed to reduce the diameter toward the longitudinal axis C more at the distal part to the tube body 152 than at the proximal part to the same.

As described above, it is preferable for the proximal end sides (the parts distal to the tube body 152) of both the inner layer 172 and the outer layer 174 in particular to be energized so that they get closer to the longitudinal axis (the central axis) C. On the other hand, the proximal end side of the extended portions 192 may be moved closer to the longitudinal axis (the central axis) C by the stretch properties of the outer layer 174 without exerting the energizing force on the extended portions 192 of the inner layer 172.

Further, it is preferable to form each extended portion 192 to be thinner on the proximal end side (the distal part to the tube body 152) than on the distal end side (the proximal part to the tube body 152). In this case, a step between the proximal end of the spiral unit 60 and the outer peripheral surface of the insertion section 12 can be formed to be smaller than that in a case where wall thicknesses of the extended portions 192 are fixed from the distal end to the proximal end.

Furthermore, the inner peripheral surfaces of the extended portions 192 on the proximal end side (distal part) to the tube body 152 can abut on the outer peripheral surface of the insertion section 12. Moreover, the spiral unit 60 rotates in the periaxial direction (the circumferential direction) of the longitudinal axis C on the outer peripheral surface of the insertion section 12. Thus, it is preferable to select a material that is slippery on the outer peripheral surface of the insertion section 12 in the periaxial direction of the longitudinal axis C for the inner peripheral surfaces of the extended portions 192 on the proximal end side.

Additionally, considering a case where the inner peripheral surface of the outer layer 174 abuts on the outer peripheral surface of the insertion section 12, it is preferable to select a material that is slippery on the outer periphery of the insertion section 12 in the periaxial direction of the longitudinal axis C for the inner peripheral surface of the outer layer 174 on the proximal end side.

As described above, the spiral unit 60 according to this embodiment is disposable. Thus, the spiral unit 60 is removed before cleaning, sanitizing, and sterilizing the endoscope 10. As a matter of course, the endoscope 10 mounting the spiral unit 60 may be disposable.

Here, the minimum inner diameter of the tube body 152 is formed to be equal to or larger than the maximum outer diameters of the distal rigid section 42, the bending section 44, the first flexible section 46, and the first relay connecting section 50 of the insertion section 12. In a state that the spiral unit 60 is disposed at a predetermined position on the outer side of the insertion section 12, the distal side taper section 156 is energized to abut in closer proximity to the longitudinal axis C on its distal end side (the distal part to the tube body 152), and the proximal side taper section 158 is energized to abut in closer proximity to the longitudinal axis C on its proximal end side (the distal part to the tube body 152). Thus, at the time of inserting the distal rigid section 42, the bending section 44, and the first flexible section 46 of the insertion section 12 into the spiral unit 60, the minimum inner diameters of the distal side taper section 156 and the proximal side taper section 158 must be adjusted to be equal to or larger than the maximum outer diameters of the distal rigid section 42, the bending section 44, the first flexible section 46, and the first relay connecting section 50.

As shown in FIG. 5A, in the proximal side taper section 158 of the spiral unit 60 is arranged a reduced diameter regulator (an expanded diameter holder) 210 that expands diameters of the respective inner peripheral surfaces of the inner layer 172 and the outer layer 174 to inner diameters that enable insertion of the distal rigid section 42, the bending section 44, and the first flexible section 46 of the insertion section 12 in advance, and regulates the inner diameters of the inner layer 172 and the outer layer 174 from being reduced.

The spiral unit 60 according to this embodiment is packaged in a state that the reduced diameter regular 210 is arranged in the proximal side taper section 158 in addition to the tube body 152 and the proximal side taper section 158. If the distal side taper section 156 has the same configuration as the proximal side taper section 158, it is preferable for each spiral unit 60 to be packaged in a state that the reduced diameter regulator 210 is arranged in not only the proximal side taper section 158 but also the distal side taper section 156.

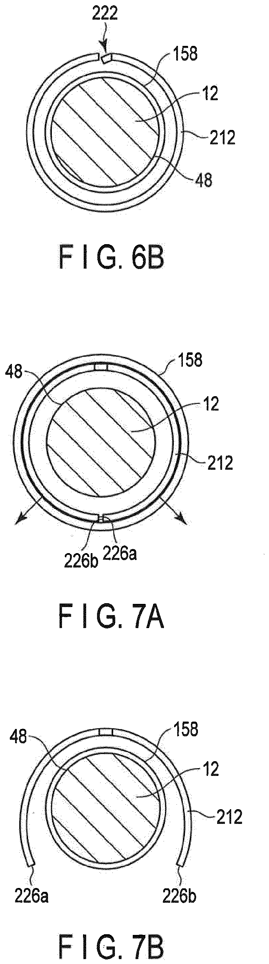

The reduced diameter regulator 210 has a ring-shaped member 212 and a finger grip 214 whose one end is fixed to the ring-shaped member 214. The finger grip 214 is extended to the proximal end side apart from the proximal end of the proximal side taper section 158. As shown in FIG. 6A and FIG. 6B, the ring-shaped member 212 of the reduced diameter regulator 210 has a portion 222 which is formed to be thinner than other regions and is easy to break (a region formed to be weaker than other regions). That is, the ring-shaped member 212 is formed so that it can be partially broken. The reduced diameter regulator 210 is removably arranged in the taper section 158 of the spiral unit 60, maintains a state that the inner diameter of the taper section 158 is expanded to allow insertion of the insertion section 12 into the taper section 158, and regulates reduction of the inner diameter of the taper section 158. Furthermore, when the reduced diameter regulator 210 is removed as the insertion section 12 is inserted into the taper section 158, it exerts a diameter reducing force to the taper section 158 more at the distal part to the tube body 152 than at the proximal part to the tube body 152 in the range where sliding is possible on the outer peripheral surface of the insertion section 12 in the periaxial direction of the longitudinal axis C.

The insertion section 12 is inserted into the tube body 152 and the proximal side taper section 158 expanding the inner diameter, the spiral unit 60 is arranged at a predetermined position on the outer side of the insertion section 12, then the finger grip 214 extended to the proximal end side of the spiral unit 60 is held, and this finger grip 214 is pulled toward the proximal end direction C2. The easy-to-break portion 222 is broken simultaneously when the finger grip 214 is pulled toward the proximal end direction C2, or the easy-to-break portion 222 can be broken after the reduced diameter regulator (the expanded diameter holder) 210 is removed from the proximal end of the spiral unit 60. A broken state of the easy-to-break portion 222 is confirmed while holding the finger grip 214, and the ring-shaped member 212 is extracted in a direction orthogonal to the longitudinal axis C of the insertion section 12. Thus, as shown in FIG. 6B, the ring-shaped member 212 can be removed from the outer side of the insertion section 12. It is to be noted that the proximal end of the proximal side taper section 158 undergoes diameter reduction to abut on the outer peripheral surface of the insertion section 12 with the removal of the ring-shaped member 212 of the reduced diameter regulator 210 from the proximal end of the proximal side taper section 158.

As another example of the reduced diameter regulator 210, the ring-shaped member 212 of the reduced diameter regulator 210 shown in FIG. 7A is formed into a C ring shape having a notch portion 226. The ring-shaped member 212 is energized to separate end portions 226a and 226b of the notch portion 226 from each other. Thus, after the insertion section 12 is inserted into the tube body 152 and the proximal side taper section 158 expanding the inner diameter, when the ring-shaped member 212 is extracted from the inner periphery of the proximal side taper section 158 while holding the finger grip 214, the end portions 226a and 226b of the notch portion 226 of the ring-shaped member 212 are separated from each other, and the ring-shaped member 212 can be removed from the outer side of the insertion section 12. It is to be noted that, with the removal of the ring-shaped member 212 of the reduced diameter regulator 210 from the proximal end of the proximal side taper section 158, the proximal end of the proximal side taper section 158 undergoes diameter reduction to abut on the outer peripheral surface of the insertion section 12.

A function of the endoscope 10 according to this embodiment will now be described.

The spiral unit 60 to be disposed on the insertion section 12 of the endoscope 10 is prepared. The reduced diameter regulators 210 are arranged on the inner peripheral surface of the distal end of the distal side taper section 156 and the inner peripheral surface of the proximal end of the proximal side taper section 158 in the spiral unit 60 respectively, and the edge portions 194a and 194b of the extended portions 192 adjacent to each other are separated from each other to expand the respective inner diameters. In this state, the distal rigid section 42, the bending section 44, and the first flexible section 46 of the insertion section 12 of the endoscope 10 are inserted into the proximal side taper section 158 of the spiral unit 60.

Further, the inner peripheral surface 182a of the annular portion 182 of the proximal side taper section 158 of the spiral unit 60 is fitted on the outer peripheral surface of the cover member 126 of the insertion section 12.

In this state, the finger grip 214 of the reduced diameter regulator 210 arranged in the proximal side taper section 158 is pulled toward the proximal end direction C2 of the longitudinal axis C, and the reduced diameter regulator 210 is thereby removed from the proximal side taper section 158. The finger grip 214 of the reduced diameter regulator 210 arranged in the distal side taper section 156 is pulled toward the distal end direction C1 of the longitudinal axis C, and the reduced diameter regulator 210 is thereby removed from the distal side taper section 156.

Thus, the edge portions 194a and 194b of the extended portions 192 adjacent to each other are allowed to abut on each other, the inner diameter is reduced more on the proximal end side of the proximal side taper section 158, and the inner diameter is reduced more on the distal end side of the distal side taper section 156. Furthermore, the spiral unit 60 is appropriately arranged at the predetermined position on the outer side of the insertion section 12. At this time, the distal end and the proximal end of the spiral unit 60 are energized toward the longitudinal axis C to maintain an abutting state on the outer peripheral surface of the insertion section 12. Thus, the step between the outer peripheral surface of the insertion section 12 and the distal end of the distal side taper section 156 and that between the outer peripheral surface of the insertion section 12 and the proximal end of the proximal side taper section 158 are minimized as much as possible. Therefore, since the distal side taper section 156 is formed so that its outer diameter is gradually reduced toward the proximal end direction C1 of the longitudinal axis C, the distal side taper section 156 forms a moderately inclined surface extending from the outer peripheral surface of the insertion section 12 to the distal end outer periphery of the tube body 152. Likewise, since the proximal side taper section 158 is formed so that its outer diameter is gradually reduced toward the proximal end direction C2 of the longitudinal axis C, the proximal side taper section 158 forms a moderately inclined surface extending from the outer peripheral surface of the insertion section 12 to the proximal end outer periphery of the tube body 152.

The insertion section 12 having the spiral unit 60 disposed thereon in this manner is inserted into a duct. In a state that the spiral fin 154 abuts on an inner wall of the duct, the motor (the drive member) 132 is driven to rotate the spiral unit 60 in the periaxial direction of the longitudinal axis C of the insertion section 12 as described above.

Specifically, in a state that the spiral fin 154 spirally extended around the longitudinal axis C receives a pressing force from the inner wall of the duct toward the longitudinal axis (the central axis) C, the spiral unit 60 is rotated in one of the periaxial directions of the longitudinal axis C. At this time, even if the distal end and the proximal end of the spiral unit 60 are appressed against the outer peripheral surface of the insertion section 12 by the energizing force, their contact force enables rotating the spiral unit 60 in one of the periaxial directions of the longitudinal axis C on the insertion section 12. When the spiral unit 60 rotates in one of the periaxial directions of the longitudinal axis C, a propulsive force in the distal end direction C1 acts on the distal end of the insertion section 12.

Furthermore, in a state that the spiral fin 154 receives the pressing force from the inner wall of the duct toward the longitudinal axis (the central axis) C, the spiral unit 60 (the tube body 152 and the spiral fin 154) is rotated in the other of the periaxial directions of the longitudinal axis C. At this time, even if the distal end and the proximal end of the spiral unit 60 are appressed against the outer peripheral surface of the insertion section 12 by an energizing force, their contact force enables rotating the spiral unit 60 in the other of the periaxial directions of the longitudinal axis C on the insertion section 12. When the spiral unit 60 rotates in the other of the periaxial directions of the longitudinal axis C, the propulsive force in the proximal end direction C2 acts on the distal end of the insertion section 12.

As described above, insertion properties of the insertion section 12 into the duct are improved by the propulsive force in the distal end direction C1, and removal properties of the insertion section 12 from the duct can be improved by the propulsive force in the proximal end direction C2.

The distal end of the distal side taper section 156 formed in the same manner as the proximal side taper section 158 is formed with a small step to the outer peripheral surface of the insertion section 12 by the above-described configuration. Furthermore, the distal side taper section 156 forms the moderately inclined surface extending from the outer peripheral surface of the insertion section 12 to the distal end outer periphery of the tube body 152. Moreover, the proximal end of the proximal side taper section 158 is formed with a small step to the outer peripheral surface of the insertion section 12 by the above-described configuration. Additionally, the proximal side taper section 158 forms the moderately inclined surface extending from the outer peripheral surface of the insertion section 12 to the proximal end outer periphery of the tube body 152. Therefore, for example, even in a region where a size of a transverse cross section of a duct precipitously changes from a large state to a small state, an inner wall of the duct can be prevented from being caught on a boundary between the distal end of the distal side taper section 156 and the outer peripheral surface of the insertion section 12 and the distal end of the tube body 152 as much as possible at the time of inserting the insertion section 12 into the duct toward the distal end direction C1. Likewise, for example, even in the region where the size of the transverse cross section of the duct precipitously changes from the large state to the small state, the inner wall of the duct can be prevented from being caught on a boundary between the proximal end of the proximal side taper section 158 and the outer peripheral surface of the insertion section 12 and the proximal end of the tube body 152 as much as possible at the time of removing the insertion section 12 from the duct toward the proximal end direction C2. Thus, in the spiral unit 60 according to this embodiment, its end portion can be prevented from being caught on the inner peripheral surface of the duct while the spiral unit 60 is appropriately disposed on the insertion section 12.

It is to be noted that, in this embodiment, the endoscope 10 having the observation optical system and the illumination optical system has been described as the introduction apparatus for various kinds of ducts. It is possible to arrange the same spiral unit 60 to a catheter which does not have both the observation optical system and the illumination optical system as the introduction apparatus for various kinds of ducts.

In the first embodiment, as shown in FIG. 2 and FIG. 3, the relay gear 116 is meshed with the drive gear 114, and the inner peripheral gear section 122 of the rotary tubular member 120 is meshed with the relay gear 116 so that the driving force from the motor 132 is transmitted to the rotary tubular member 120. Of these members, the relay gear 116 is not necessarily required. That is, it is also preferable to form the members so that the driving force can be directly transmitted from the drive gear 114 to the inner peripheral gear section 122 of the rotary tubular member 120.

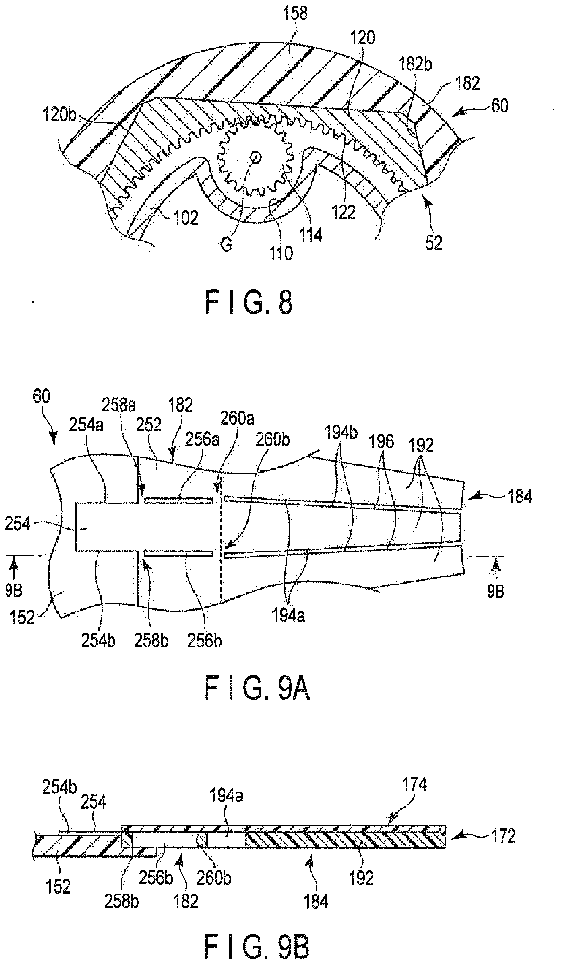

Furthermore, in the first embodiment, as shown in FIG. 2 to FIG. 4, the description has been given as to the example where one inner roller 124a is arranged between the two outer rollers 162a and 162b in three pairs. In an example shown in FIG. 8, these rollers 124a to 124c and 162a to 162f are eliminated. In the example shown in FIG. 8, the outer peripheral surface 120b of the rotary tubular member 120 and the inner peripheral surface 182b of the annular portion 182 of the proximal side taper section 158 of the spiral unit 60 are formed into shapes that can be fitted to each other. Therefore, when the outer peripheral surface 120b of the rotary tubular member 120 and the inner peripheral surface 182b of the annular portion 182 of the proximal side taper section 158 of the spiral unit 60 are fitted to each other, the driving force of the motor 132 can be transmitted from the drive gear 114 to the inner peripheral gear section 122 of the rotary tubular member 120 to rotate the spiral unit 60 in the periaxial direction of the longitudinal axis C.

A description will now be given as to a mechanism that releases diameter reduced states of the distal side taper section 156 and the proximal side taper section 158 to remove the spiral unit 60 from the outer periphery of the insertion section 12 of the endoscope 10 according to this embodiment. That is, the spiral unit 60 according to this embodiment has a removal mechanism that removes the spiral unit 60 from the outer peripheral surface of the insertion section 12 of the endoscope 10 and discards it. Here, although an example where the removal mechanism is arranged to the proximal side taper section 158 will be described, arranging the same mechanism to the distal side taper section 156 is also preferable.

As shown in FIG. 9A and FIG. 9B, the annular section 182 of the inner layer 172 has an annular main body 252, a tab 254 extended from the annular main body 252 to the distal end side (the tube main body 152 side), and a pair of slits 256a and 256b formed in the annular main body 252. The tab 254 is formed to aid removal of the proximal side taper section 158 so that the insertion section 12 can be released from a state that a diameter reducing force is exerted to enable sliding on the outer peripheral surface of the insertion section 12 on the proximal end side of the tube body 152 in the periaxial direction of the longitudinal axis C.

Distal ends of the slits 256a and 256b are formed near the tab 254. A width between the slits 256a and 256b is formed to be substantially equal to a circumferential width of a base part of the extended portion 192 integral with the annular portion 182. One edge portion 194a of a given extended portion 192, the slit 256b, and one edge portion 254b of the tab 254 are provided on a substantially straight line, and the other edge portion 194b of the given extended portion 192, the slit 256a, and the other edge portion 254a of the tab 254 are provided on a substantially straight line. A back surface of the tab 254 is usually attached to the outer peripheral surface of the tube body 152.

Thus, when the tab 254 is lifted up to the outer peripheral surface of the tube body 152 and a force is added to the proximal end side, stress is concentrated on regions 258a and 258b between the edge portions 254a and 254b of the tab 254 and the slits 256a and 256b respectively, and the regions on which the stress is concentrated are broken. At this time, the outer layer 174 appressed against the outer side of the inner layer 172 is torn up together. Moreover, stress is concentrated on regions 260a and 260b between the slits 256a and 256b and the edge portions 194a and 194b of a given extended portion 192 respectively, and the regions on which the stress is concentrated are broken.

When the inner layer 172 and the outer layer 174 are torn up in this manner, the diameter reducing force (contraction force) does not work on the inner layer 172 and the outer layer 174. Thus, the spiral unit 60 including the distal side taper section 156 and the proximal side taper section 158 that have lost the diameter reducing force can be moved to the distal end direction C1 on the insertion section 12. Therefore, the spiral unit 60 can be easily removed from the outer side of the insertion section 12.

In addition, the inner peripheral surfaces of the proximal ends of the extended portions 192 abut on the outer peripheral surface of the second flexible section 48 in FIG. 2, but it is also preferable to extend the relay member 106 in the proximal end direction C2 so that the inner peripheral surfaces of the proximal ends of the extended portions 192 can preferably abut on the outer peripheral surface of the relay member 106.

Additionally, the distal end of the distal side taper section 156 may be preferably formed to abut on the outer peripheral surface of the bending section 44, or may be preferably formed to abut on the outer peripheral surface of the first relay connecting section 50.

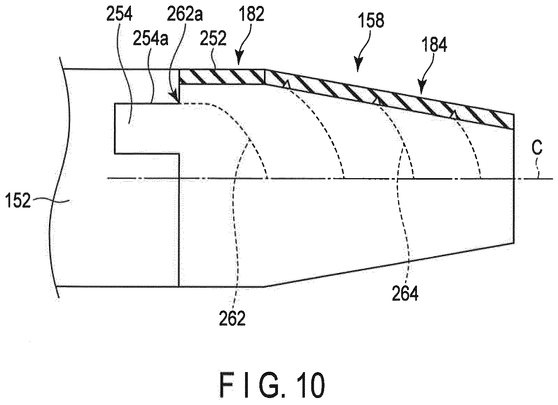

A second embodiment will now be described with reference to FIG. 10. This embodiment is a modification of the first embodiment, like reference numerals denote the same members or members having the same functions as those described in the first embodiment, thereby omitting a detailed description thereof.

A proximal side taper section 158 of a spiral unit 60 according to this embodiment shown in FIG. 10 is formed of a single layer. The proximal side taper section 158 is formed of an elastic member to exert an energizing force so that the proximal side taper section 158 can approximate a longitudinal axis C on its proximal end side. A diameter of the proximal side taper section 158 can be reduced more at a distal part to a tube body 152 toward the longitudinal axis C than at a proximal part to the tube body 152. Even if the proximal side taper section 158 is formed in this manner, it can function in the same manner as the proximal side taper section 158 having the inner layer 172 and the outer layer 174 described in the first embodiment.

The proximal side taper section 158 according to this embodiment has an annular portion 182 coupled with a proximal end of the tube body 152, and a deforming portion 184 which is integrally formed with a proximal end of the annular portion 182 and formed into a truncated conical shape so that an inner diameter and an outer diameter are reduced toward a proximal end side. It is preferable to form the annular portion 182 in the same manner as the annular portion 182 described in the first embodiment. The deforming portion 184 can be expanded to the longitudinal axis C along a radial direction.

The annular portion 182 of the proximal side taper section 158 according to this embodiment has a main body 252, a tab 254 extended from the annular main body 252 to a distal end side (the tube body 152 side), and a groove (a thin wall portion) 262 formed on an inner peripheral surface of the annular main body 252. It is preferable to form the groove 262 into, e.g., a helical shape.

On an inner peripheral surface of the deforming portion 184, a groove (a thin wall portion) 264 is formed continuously with the groove (the thin wall portion) 262 formed on the inner peripheral surface of the annular main body 252. It is also preferable to form this groove 264 into, e.g., a helical shape.

It is to be noted that the groove 264 on the deforming portion 184 is formed while setting a material or a depth thereof to avoid damage at the time of expanding the deforming portion 184 to the longitudinal axis C along the radial direction so that the insertion section 12 can be inserted. Likewise, the groove 262 formed on the main body 252 of the annular portion 182 is formed while setting a material or a depth thereof to avoid damage caused due to an influence at the time of expanding the deforming portion 184 to the longitudinal axis C along the radial direction so that the insertion section 12 can be inserted.

It is preferable to form not only the proximal side taper section 158 but also a distal side taper section 156 in the same manner.

Thus, the spiral unit 60 according to this embodiment can be used in the same manner as the spiral unit 60 described in the first embodiment. That is, the spiral unit 60 according to this embodiment is packaged in a state that a reduced diameter regulator (an expanded diameter holder) 210 is arranged at each of the distal end and the proximal end. Further, at the time of disposing the spiral unit 60 at an appropriate position on the outer periphery of the insertion section 12, the spiral unit 60 can be disposed on the outer side of the insertion section 12 by removing the reduced diameter regulator (the expanded diameter holder) 210. In a state that the spiral unit 60 is appropriately arranged at the predetermined position on the outer side of the insertion section 12, the distal end and the proximal end of the spiral unit 60 are energized toward the longitudinal axis C to maintain a state that the distal end and the proximal end abut on the outer peripheral surface of the insertion section 12, respectively. Thus, a step between the outer peripheral surface of the insertion section 12 and the distal end of the distal side taper section 156 and that between the outer peripheral surface of the insertion section 12 and the proximal end of the proximal side taper section 158 are minimized as much as possible. Therefore, for example, even in a region where a size of a transverse cross section of a duct precipitously changes from a large state to a small state, an inner wall of the duct can be prevented from being caught on a boundary between the distal end of the distal side taper section 156 and the outer peripheral surface of the insertion section 12 as much as possible at the time of inserting the insertion section 12 into the duct toward a distal end direction C1. Likewise, for example, even in the region where the size of the transverse cross section of the duct precipitously changes from the large state to the small state, the inner wall of the duct can be prevented from being caught on a boundary between the proximal end of the proximal side taper section 158 and the outer peripheral surface of the insertion section 12 as much as possible at the time of removing the insertion section 12 from the duct toward a proximal end direction C2. Thus, in the spiral unit 60 according to this embodiment, its end portion can be prevented from being caught on the inner peripheral surface of the duct while the spiral unit 60 is appropriately disposed on the insertion section 12.

A brief description will now be given as to a function at the time of releasing diameter reduced states of the distal side taper section 156 and the proximal side taper section 158 to remove the spiral unit 60 according to this embodiment from the outer periphery of the insertion section 12 of the endoscope 10.

When the tab 254 is lifted up to the outer peripheral surface of the tube body 152 and force is added to the proximal end side, stress is concentrated on a region between an edge portion 254a of the tab 254 and the groove 262, namely, a distal end 262a of the groove 262, and breakage occurs from the region on which the stress is concentrated along the groove 262. That is, the main body 252 of the annular portion 182 is broken and delaminated from the distal end toward the proximal end of the groove 262. Furthermore, the groove 264 on the deforming portion 184 continuous with the groove 262 on the main body 252 of the annular portion 182 is likewise broken and delaminated from the distal end toward the proximal end.

When the main body 252 of the annular portion 182 is torn up along the groove 262 and the deforming portion 184 is torn up along the groove 264 in this manner, the diameter reducing force (contraction force) does not work on the proximal side taper section 158 formed of the elastic member.

In the case of releasing the diameter reduced state of the distal side taper section 156, the same operation as that for the proximal side taper section 158 could be performed.

Thus, the spiral unit 60 including the distal side taper section 156 and the proximal side taper section 158 that have lost the diameter reducing force can be moved to the distal end direction C1 on the insertion section 12. Therefore, the spiral unit 60 can be easily removed from the outer side of the insertion section 12.

Although the description has been given as to the case where the proximal side taper section 158 is the single layer in this embodiment, it is preferable to cover the outer periphery of the proximal side taper section 158 according to this embodiment with the outer layer 174 described in the first embodiment. In this case, it is possible to more assuredly prevent the regions (the thin wall portions) 258a, 258b, 260a, and 260b adjacent to the slits 256a and 256b of the proximal side taper section 158 from being damaged during the use of the endoscope 10.

In the spiral unit 60, for example, a taper section having the configuration described in the first embodiment may be adopted for the distal side taper section 156, and a taper section having the configuration described in the second embodiment may be adopted for the proximal side taper section 158. Additionally, for example, the taper section having the configuration described in the second embodiment may be adopted for the distal side taper section 156, and the taper section having the configuration described in the first embodiment may be adopted for the proximal side taper section 158.

Although several embodiments have been specifically described with reference to the drawings, the present invention is not restricted to the foregoing embodiments.

## Claims

1. A spiral unit (60) into which an insertion section (12) of an introduction apparatus (10) having a longitudinal axis (C) is to be inserted, and which is rotatable relative to the insertion section (12) in a state of being disposed on the insertion section (12), the spiral unit (60) comprising:
a tube body (152) which is configured to be arranged along the longitudinal axis (C), and has a spiral fin (154) arranged on an outer peripheral surface thereof; and
a taper section (158) with a tubular shape which is provided on at least one of a distal end side and a proximal end side of the tube body (152) along the longitudinal axis (C), has a proximal part close to the tube body (152) and a distal part apart from the tube body (152), has an outer periphery that is diameter-reduced toward the longitudinal axis (C) more at the distal part than at the proximal part along the longitudinal axis (C), and is configured to exert an inner diameter reducing force to an outer peripheral surface of the insertion section (12) at the distal part in the range where sliding is possible in a periaxial direction of the longitudinal axis (C);
**characterized in that** the spiral unit (60) further comprises:
an expanded diameter holder (210) which is removably arranged in the taper section (158), and configured to maintain a state that the inner diameter of the taper section (158) is expanded to allow insertion of the insertion section (12) into the taper section (158) and to regulate reduction of the inner diameter of the taper section (158), wherein the taper section (158) is configured to exert the inner diameter reducing force when the expanded diameter holder (210) is removed and more at the distal part than the proximal part.

2. The spiral unit (60) according to claim 1, wherein the expanded diameter holder (210) is configured to be removable from the taper section (158) in a state that the insertion section (12) is inserted into the taper section (158).

3. The spiral unit (60) according to claim 1, wherein
the taper section (158) comprises:
an inner layer (172) having strip-shaped extended portions (192) which are aligned in a circumferential direction of the longitudinal axis (C) and extended from the proximal part to the tube body (152) to the distal part to the tube body (152); and
a tubular outer layer (174) which covers an outer side of the inner layer (174), and reduces a diameter of the extended portions (192) toward the longitudinal axis (C) more at the distal part to the tube body (152) than at the proximal part to the tube body (152).

4. The spiral unit (60) according to claim 3, wherein the extended portions (192) themselves are formed to reduce the diameter toward the longitudinal axis (C) more at the distal part to the tube body (152) than at the proximal part to the tube body (152).

5. The spiral unit (60) according to claim 3, wherein
each of the extended portions (192) has a pair of edge portions (194a), and
the extended portions (192) are formed to reduce the diameter at the distal part to the tube body (152) by allowing the edge portions (192) of the adjacent extended portions (192) in the extended portions (102) to abut on each other, and to expand the diameter at the distal part to the tube body (152) by separating the edge portions (194a) of the adjacent extended portions (192) from each other.

6. The spiral unit (60) according to claim 3, wherein the inner layer (172) has a tab (254) which aids removal of the taper section (158) to release the insertion section (12) from a state that the diameter reducing force is exerted in the range where sliding is possible on the outer peripheral surface of the insertion section (12) in the periaxial direction of the longitudinal axis (C) at the distal part to the tube body (152).

7. The spiral unit (60) according to claim 1, wherein the taper section (158) is formed into a single layer by using an elastic member (184) to reduce the diameter toward the longitudinal axis (C) more at the distal part to the tube body (152) than at the proximal part to the tube body (152).

8. The spiral unit (60) according to claim 7, wherein a diameter of the elastic member (184) is expandably reduced at the distal part to the tube body (152).

9. The spiral unit (69) according to claim 7, wherein the taper section (158) has a tab (258) which aids removal of the taper section (158) to enable release of the insertion section (12) from a state that the diameter reducing force is exerted in the range where sliding is possible on the outer peripheral surface of the insertion section (12) in the periaxial direction of the longitudinal axis (C) at the distal part to the tube body (152).

10. The spiral unit (60) according to claim 9, wherein the taper section (158) has, on its inner peripheral surface, a groove (262) which is continuous from the tab (258) and along which the taper section (158) is delaminated from the outer side of the insertion section (12).

11. The spiral unit (60) according to claim 1, wherein the expanded diameter holder (210) has a ring-shaped member (212) formed in such a manner that it can be partially broken.

12. The spiral unit (60) according to claim 1, wherein the expanded diameter holder (210) has a ring-shaped member (212) which has a notch portion (226) and is energized so that end portions (226, 226b) thereof are separated from each other, when the ring-shaped member (212) is extracted from the inner periphery of the taper section (158).

13. The spiral unit (60) according to claim 1, comprising: on its inner peripheral surface, a roller (124a) which is engaged to transmit a rotational force of the insertion section (12) around the longitudinal axis (C) to the tube body (154) in state that the insertion section (12) having the longitudinal axis (C) is inserted.

14. An introduction apparatus (10) for various kinds of ducts, comprising:
an insertion section (12) which is extended along a longitudinal axis (C), and inserted into a duct from a distal end thereof; and
a spiral unit (60) according to claim 1 which is disposed on an outer periphery of the insertion section (12), and rotatable in a periaxial direction of the longitudinal axis (C) relative to the insertion section (12) by driving of a drive member (132).

## Patentansprüche

1. Spiraleinheit (60), in die ein Einführabschnitt (12) einer Einführvorrichtung (10) mit einer Längsachse (C) einzuführen ist, und die relativ zum Einführabschnitt (12) in einem Zustand drehbar ist, in dem sie an dem Einführabschnitt (12) angeordnet ist, wobei die Spiraleinheit (60) umfasst:
einen Röhrenkörper (152), der dazu ausgelegt ist, entlang der Längsachse (C) angeordnet zu sein, und der eine spiralförmige Lamelle (154) aufweist, die auf einer Außenperipheriefläche davon angeordnet ist; und
einen Konusabschnitt (158) mit einer röhrenförmigen Form, der auf mindestens einer von einer Distalendseite und einer Proximalendseite des Röhrenkörpers (152) entlang der Längsachse (C) vorgesehen ist, einen proximalen Teil nahe dem Röhrenkörper (152) und einen distalen Teil abseits des Röhrenkörpers (152) aufweist, eine Außenperipherie aufweist, die in Richtung der Längsachse (C) eher an dem distalen Teil als an dem proximalen Teil entlang der Längsachse (C) im Durchmesser reduziert ist, und der dazu ausgelegt ist, eine den Innendurchmesser reduzierende Kraft auf eine Außenperipheriefläche des Einführabschnitts (12) an dem distalen Teil in dem Bereich auszuüben, in dem das Gleiten in einer periaxialen Richtung der Längsachse (C) möglich ist;
**dadurch gekennzeichnet, dass** die Spiraleinheit (60) ferner umfasst:
einen Durchmesseraufweiter (210), der in dem Konusabschnitt (158) entfernbar angeordnet und dazu ausgelegt ist, einen Zustand beizubehalten, in dem der Innendurchmesser des Konusabschnitts (158) aufgeweitet ist, um das Einführen des Einführabschnitts (12) in den Konusabschnitt (158) zu erlauben und die Reduktion des Innendurchmessers des Konusabschnitts (158) zu regulieren, wobei der Konusabschnitt (158) dazu ausgelegt ist, die den Innendurchmesser reduzierende Kraft auszuüben, wenn der Durchmesseraufweiter (210) eher an dem distalen Teil als dem proximalen Teil entfernt wird.

2. Spiraleinheit (60) nach Anspruch 1, wobei der Durchmesseraufweiter (210) dazu ausgelegt ist, in einem Zustand aus dem Konusabschnitt (158) entfernt werden zu können, in dem der Einführabschnitt (12) in den Konusabschnitt (158) eingeführt ist.

3. Spiraleinheit (60) nach Anspruch 1, wobei der Konusabschnitt (158) umfasst: eine Innenschicht (172) mit streifenförmigen verlängerten Abschnitten (192), die in einer Umlaufrichtung der Längsachse (C) ausgerichtet sind und sich von dem proximalen Teil des Röhrenkörpers (152) bis zu dem distalen Teil des Röhrenkörpers (152) erstrecken; und
eine röhrenförmige Außenschicht (174), die eine Außenseite der Innenschicht (174) abdeckt und einen Durchmesser der verlängerten Abschnitte (192) in Richtung der Längsachse (C) eher an dem distalen Teil des Röhrenkörpers (152) als an dem proximalen Teil des Röhrenkörpers (152) reduziert.

4. Spiraleinheit (60) nach Anspruch 3, wobei die verlängerten Abschnitte (192) selbst dazu gebildet sind, den Durchmesser in Richtung der Längsachse (C) eher an dem distalen Teil des Röhrenkörpers (152) als an dem proximalen Teil des Röhrenkörpers (152) zu reduzieren.

5. Spiraleinheit (60) nach Anspruch 3, wobei
jeder der verlängerten Abschnitte (192) ein Paar Randabschnitte (194a) aufweist, und
die verlängerten Abschnitte (192) dazu gebildet sind, den Durchmesser an dem distalen Teil des Röhrenkörpers (152) durch Zulassen, dass die Randabschnitte (192) der nebeneinanderliegenden verlängerten Abschnitte (192) in den verlängerten Abschnitten (102) aneinandergrenzen, zu reduzieren, und den Durchmesser an dem distalen Teil des Röhrenkörpers (152) durch Voneinandertrennen der Randabschnitte (194a) der nebeneinanderliegenden verlängerten Abschnitte (192) zu erweitern.

6. Spiraleinheit (60) nach Anspruch 3, wobei die Innenschicht (172) eine Lasche (254) aufweist, die das Entfernen des Konusabschnitts (158) unterstützt, um den Einführabschnitt (12) aus einem Zustand freizugeben, in dem die den Durchmesser reduzierende Kraft in dem Bereich ausgeübt wird, in dem das Schieben auf der Außenperipheriefläche des Einführabschnitts (12) in der periaxialen Richtung der Längsachse (C) an dem distalen Teil des Röhrenkörpers (152) möglich ist.

7. Spiraleinheit (60) nach Anspruch 1, wobei der Konusabschnitt (158) in einer einzelnen Schicht mithilfe eines elastischen Elements (184) gebildet ist, um den Durchmesser in Richtung der Längsachse (C) eher an dem distalen Teil des Röhrenkörpers (152) als an dem proximalen Teil des Röhrenkörpers (152) zu reduzieren.

8. Spiraleinheit (60) nach Anspruch 7, wobei ein Durchmesser des elastischen Elements (184) an dem distalen Teil des Röhrenkörpers (152) weiter reduziert wird.

9. Spiraleinheit (69) nach Anspruch 7, wobei der Konusabschnitt (158) eine Lasche (258) aufweist, die das Entfernen des Konusabschnitts (158) unterstützt, um den Einführabschnitt (12) aus einem Zustand freizugeben, in dem die den Durchmesser reduzierende Kraft in dem Bereich ausgeübt wird, in dem das Schieben auf der Außenperipheriefläche des Einführabschnitts (12) in der periaxialen Richtung der Längsachse (C) an dem distalen Teil des Röhrenkörpers (152) möglich ist.

10. Spiraleinheit (60) nach Anspruch 9, wobei der Konusabschnitt (158) auf seiner Innenperipheriefläche eine Nut (262) aufweist, die sich kontinuierlich von der Lasche (258) erstreckt und entlang welcher der Konusabschnitt (158) von der Außenseite des Einführabschnitts (12) delaminiert wird.

11. Spiraleinheit (60) nach Anspruch 1, wobei der Durchmesseraufweiter (210) ein ringförmiges Element (212) aufweist, das derart gebildet ist, dass es teilweise aufgebrochen werden kann.

12. Spiraleinheit (60) nach Anspruch 1, wobei der Durchmesseraufweiter (210) ein ringförmiges Element (212) aufweist, das einen Kerbabschnitt (226) aufweist und derart energetisiert wird, dass die Endabschnitte (226, 226b) davon voneinander getrennt sind, wenn das ringförmige Element (212) aus der Innenperipherie des Konusabschnitts (158) entnommen wird.

13. Spiraleinheit (60) nach Anspruch 1, umfassend:
auf ihrer Innenperipheriefläche eine Rolle (124a), die im Eingriff ist, um eine Rotationskraft des Einführabschnitts (12) rund um die Längsachse (C) in einem Zustand an den Röhrenkörper (154) zu senden, in dem bei dem Einführabschnitt (12) die Längsachse (C) eingeführt ist.

14. Einführvorrichtung (10) für verschiedene Arten von Kanälen, umfassend:
einen Einführabschnitt (12), der sich entlang einer Längsachse (C) erstreckt und von einem distalen Ende davon in einen Kanal eingeführt wird; und
eine Spiraleinheit (60) nach Anspruch 1, die an einer Außenperipherie des Einführabschnitts (12) angeordnet ist und in einer periaxialen Richtung der Längsachse (C) relativ zu dem Einführabschnitt (12) durch Antreiben eines Antriebselements (132) rotiert werden kann.

## Revendications

1. Unité spiralée (60) dans laquelle une section d'insertion (12) d'un appareil d'introduction (10) ayant un axe longitudinal (C) doit être insérée, et qui est apte à tourner par rapport à la section d'insertion (12) dans un état où elle est disposée sur la section d'insertion (12), l'unité spiralée (60) comprenant :
un corps de tube (152) qui est configuré pour être disposé selon l'axe longitudinal (C), et a une ailette spiralée (154) disposée sur une surface périphérique externe de celui-ci ; et
une section effilée (158) avec une forme tubulaire qui est prévue sur au moins un parmi un côté extrémité distale et un côté extrémité proximale du corps de tube (152) selon l'axe longitudinal (C), a une partie proximale proche du corps de tube (152) et une partie distale à distance du corps de tube (152), a une périphérie externe qui est réduite en diamètre vers l'axe longitudinal (C) davantage au niveau de la partie distale qu'au niveau de la partie proximale selon l'axe longitudinal (C), et est configurée pour exercer une force de réduction de diamètre interne sur une surface périphérique externe de la section d'insertion (12) au niveau de la partie distale dans la plage dans laquelle un glissement est possible dans une direction périaxiale de l'axe longitudinal (C) ;
**caractérisée par le fait que** l'unité spiralée (60) comprend en outre :
un organe de maintien de diamètre élargi (210) qui est disposé de manière amovible dans la section effilée (158), et configuré pour maintenir un état dans lequel le diamètre interne de la section effilée (158) est élargi pour permettre l'insertion de la section d'insertion (12) dans la section effilée (158) et pour réguler la réduction du diamètre interne de la section effilée (158), la section effilée (158) étant configurée pour exercer la force de réduction de diamètre interne lorsque l'organe de maintien de diamètre élargi (210) est retiré et se situe davantage au niveau de la partie distale que de la partie proximale.

2. Unité spiralée (60) selon la revendication 1, dans laquelle l'organe de maintien de diamètre élargi (210) est configuré pour être amovible de la section effilée (158) dans un état dans lequel la section d'insertion (12) est insérée dans la section effilée (158).

3. Unité spiralée (60) selon la revendication 1, dans laquelle la section effilée (158) comprend :
une couche interne (172) ayant des parties étendues en forme de bande (192) qui sont alignées dans une direction circonférentielle de l'axe longitudinal (C) et s'étendent de la partie proximale au corps de tube (152) à la partie distale au corps de tube (152) ; et
une couche externe tubulaire (174) qui recouvre un côté externe de la couche interne (174), et qui réduit un diamètre des parties étendues (192) vers l'axe longitudinal (C) davantage au niveau de la partie distale au corps de tube (152) qu'au niveau de la partie proximale au corps de tube (152).

4. Unité spiralée (60) selon la revendication 3, dans laquelle les parties étendues (192) elles-mêmes sont formées pour réduire le diamètre vers l'axe longitudinal (C) davantage au niveau de la partie distale au corps de tube (152) qu'au niveau de la partie proximale au corps de tube (152).

5. Unité spiralée (60) selon la revendication 3, dans laquelle :
chacune des parties étendues (192) a une paire de parties de bord (194a), et
les parties étendues (192) sont formées pour réduire le diamètre au niveau de la partie distale au corps de tube (152) en permettant aux parties de bord (192) des parties étendues adjacentes (192) dans les parties étendues (102) de venir en butée les unes contre les autres, et pour élargir le diamètre au niveau de la partie distale au corps de tube (152) en séparant les parties de bord (194a) des parties étendues adjacentes (192) les unes des autres.

6. Unité spiralée (60) selon la revendication 3, dans laquelle la couche interne (172) a une languette (254) qui aide au retrait de la section effilée (158) pour libérer la section d'insertion (12) à partir d'un état dans lequel la force de réduction de diamètre est exercée dans la plage dans laquelle un glissement est possible sur la surface périphérique externe de la section d'insertion (12) dans la direction périaxiale de l'axe longitudinal (C) au niveau de la partie distale au corps de tube (152).

7. Unité spiralée (60) selon la revendication 1, dans laquelle la section effilée (158) est formée en une unique couche en utilisant un élément élastique (184) pour réduire le diamètre vers l'axe longitudinal (C) davantage au niveau de la partie distale au corps de tube (152) qu'au niveau de la partie proximale au corps de tube (152).

8. Unité spiralée (60) selon la revendication 7, dans laquelle un diamètre de l'élément élastique (184) est réduit de manière élargissable au niveau de la partie distale au corps de tube (152).

9. Unité spiralée (60) selon la revendication 7, dans laquelle la section effilée (158) a une languette (258) qui aide au retrait de la section effilée (158) pour permettre la libération de la section d'insertion (12) à partir d'un état dans lequel la force de réduction de diamètre est exercée dans la plage dans laquelle un glissement est possible sur la surface périphérique externe de la section d'insertion (12) dans la direction périaxiale de l'axe longitudinal (C) au niveau de la partie distale au corps de tube (152).

10. Unité spiralée (60) selon la revendication 9, dans laquelle la section effilée (158) a, sur sa surface périphérique interne, une rainure (262) qui est continue à partir de la languette (258) et le long de laquelle la section effilée (158) est délaminée à partir du côté externe de la section d'insertion (12).

11. Unité spiralée (60) selon la revendication 1, dans laquelle l'organe de maintien de diamètre élargi (210) a un élément en forme d'anneau (212) formé d'une manière telle qu'il peut être partiellement rompu.

12. Unité spiralée (60) selon la revendication 1, dans laquelle l'organe de maintien de diamètre élargi (210) a un élément en forme d'anneau (212) qui a une partie encoche (226) et s'est vu donner de l'énergie de telle sorte que des parties d'extrémité (226, 226b) de celui-ci sont séparées l'une de l'autre, lorsque l'élément en forme d'anneau (212) est extrait de la périphérie interne de la section effilée (158).

13. Unité spiralée (60) selon la revendication 1, comprenant :
sur sa surface périphérique interne, un rouleau (124a) qui est engagé pour transmettre une force de rotation de la section d'insertion (12) autour de l'axe longitudinal (C) au corps de tube (154) dans un état dans lequel la section d'insertion (12) ayant l'axe longitudinal (C) est insérée.

14. Appareil d'introduction (10) pour divers types de conduits, comprenant :
une section d'insertion (12) qui est étendue selon un axe longitudinal (C), et insérée dans un conduit à partir d'une extrémité distale de celui-ci ; et
une unité spiralée (60) selon la revendication 1 qui est disposée sur une périphérie externe de la section d'insertion (12), et est apte à tourner dans une direction périaxiale de l'axe longitudinal (C) par rapport à la section d'insertion (12) par entraînement d'un élément d'entraînement (132).
